# EUROPEAN PATENT APPLICATION

(11) **EP 0 579 384 A1**
(43) Date of publication of application: **19.01.1994**
(21) Application number: 93304663.3
(22) Date of filing: 15.06.1993
(51) Int. Cl.: A61M 16/08

(54) **Tubing and humidification systems**

(30) Priority: 16.07.1992 GB 9215154
(71) Applicant: Smiths Industries Public Limited Company, London, NW11 8DS (GB)
(72) Inventor: Turner, Mark William, Folkestone, Kent CT20 2RT (GB)
(74) Representative: Flint, Jonathan McNeill

(57) **Abstract**

Tubing (3) for connecting a humidifier (20) to a tracheal tube (1) or face mask has an inner corrugated tube (32) and an outer flexible tube (34) extending along its length. The outer tube (34) is bonded to the corrugated tube (32) where it contacts the corrugations (33) so that air is trapped between the corrugations. This insulates the tubing and reduces condensation.

## Description

This invention relates to tubing of the kind having a first end adapted for connection to humidifier means and a second end adapted for connection to a patient breathing device such as a tube or face mask, the tubing comprising a tube that is corrugated externally.

Where patients are ventilated or given gaseous anaesthesia, such as during surgery, it is common practice to humidify the administered gas so as to reduce the risk of damage or discomfort to the lining of the trachea. The gas can also be warmed.

The humidification can be of the passive or active kind. With passive humidification, a heat and moisture exchange device (HME) is connected into the gas-flow line. As the patient exhales, at least some of the heat and moisture in his breath is taken up by an exchange element, which may be of treated paper or foam. When the patient inhales, dry, cool gas passes through the HME in the opposite direction so that some at least of the heat and moisture retained by the element is transferred to the inhaled gas. An example of an HME is described in GB 2233904A.

Active humidifiers have water in a reservoir which is supplied to the inhaled gas such as in the form of a nebulised spray or a vapour.

The humidifying effect of an active humidifier is generally greater than that of an HME although the performance of an HME can be improved by including a heater and water supply in the manner described in WO 9119527.

The humidifier is commonly connected to a tracheal tube or face mask by means of a length of corrugated tubing. This tubing provides flexibility with a low risk of occlusion when the tubing is bent.

One problem that can occur with humidification systems, especially in those where there is a relatively high humidification effect, is that, when the warm damp gas flowing out of the humidifier comes into contact with the cool tubing, it can lead to condensation and the build-up of water. This water either collects in the tubing or runs into the humidifier or patient.

It is an object of the present invention to provide tubing and a humidifier system in which there is a reduced risk of condensation.

According to one aspect of the present invention there is provided tubing of the above-specified kind, characterised in that the tubing includes an outer tube that extends about the corrugated inner tube to trap air between the corrugations between the inner and outer tubes and thereby thermally insulate the tubing to reduce condensation of humidified gas flowing along the inner tube.

The inner tube is preferably corrugated both externally and internally and the corrugations are preferably annular so that air trapped in recesses between adjacent pairs of corrugations is isolated from each other. The outer tube is preferably uncorrugated. The inner and outer tubes are preferably transparent. The outer tube is preferably bonded to the inner tube where it contacts the corrugations.

A humidification system including tubing and connected to a ventilator, in accordance with the present invention, will now be described, by way of example, with reference to the accompanying drawings, in which:
- Figure 1: is a side elevation of the humidification system and ventilator;
- Figure 2: is an enlarged sectional side elevation view of one end of the tubing; and
- Figures 3: are enlarged sectional side and 4 elevation views of alternative forms of tubing.

The humidification system comprises a tracheal tube 1, a humidifier 2 and flexible tubing 3 linking one side of the humidifier to the tracheal tube. The other side of the humidifier 2 is connected to a ventilator 4 in the usually way.

The tubing 3 has an overall length of about 14cm and has female couplings 30 and 31 at opposite ends, which fit respectively on a tracheal tube connector 10 and on an outlet coupling 20 of the humifidier 2. The couplings 30 and 31 are formed integrally at opposite ends of an inner tube 32 of circular section which is of a transparent PVC and is formed by blow moulding or by extrusion blow moulding. Between the couplings 30 and 31, the inner tube 32 is formed with a series of annular corrugations 33 which give both the inside and outside surfaces of the tube a corrugated profile. The wall thickness of the inner tube is about 0.5mm, the external diameter of the tube at the peak of the corrugations being about 20mm and at the valley of the corrugations being about 13mm. The pitch p between adjacent corrugations is about 5mm. The tubing 3 also includes an outer transparent PVC tube 34 which is smooth in its natural state and has a wall thickness of about 0.2mm. The internal diameter of the outer tube 34 is the same as the external diameter of the inner tube 32 at the peaks of the corrugations so that the outer tube is a close sliding fit on the inner tube. The length of the outer tube 34 is just greater than the distance between the final corrugations on the inner tube so that it extends beyond the corrugations at both ends by a short distance. The outer and inner tubes 34 and 32 may be bonded together where they contact at the peaks of the corrugations, such as by a solvent or adhesive. Alternatively, the two tubes can be heat treated, such as during a sterilization procedure, to produce bonding between them.

It can be seen that the outer tube 34 traps a layer of insulating air around the inner tube 32, this layer being formed of non-communicating annular air pockets 35 in the valleys between the corrugations 33. The air pockets 35 insulate the wall of the tubing 3 so that its inside surface is maintained at a temperature close to that of the gas flowing along it. In this way, the amount of condensation produced in the tubing is substantially reduced. Although the very peaks of the corrugations of the inner tube are not insulated by air pockets, these contribute only a relatively small proportion to the overall internal surface area of the tubing. One important advantage of the insulation described above is that the tubing remains transparent, so that if a blockage or water built-up should occur in the tubing, this is readily visible to the clinician.

Both the inner and outer tubes 32 and 34 are flexible so that the tubing 3 as a whole is flexible. Bending the tubing 3 will cause a crumpling of the outer tube 34 on the inside of the bend but this does not adversely affect performance.

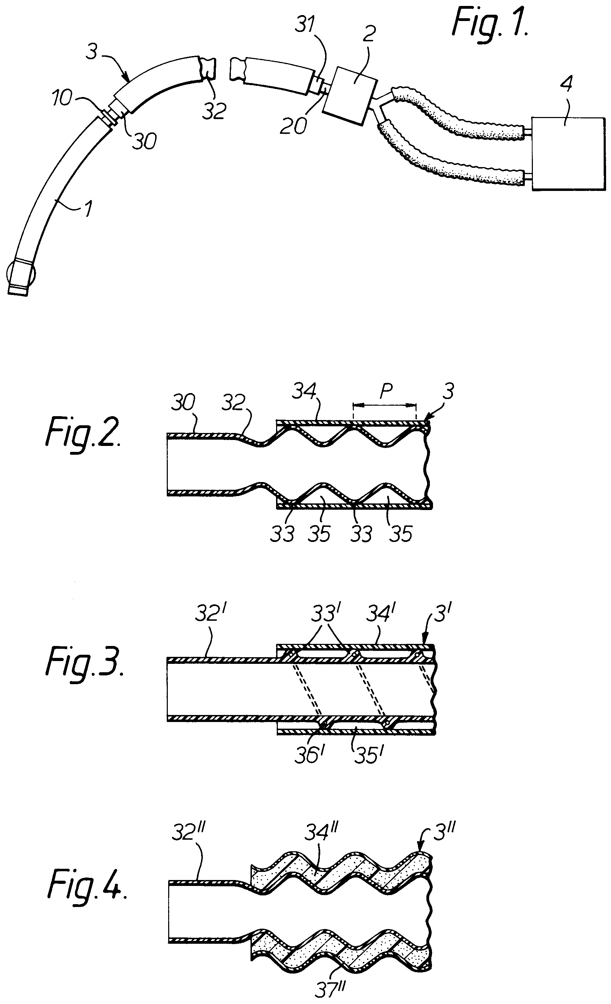

Various alternative arrangements are possible. For example, as shown in Figure 3, the inner tube 32' could be smooth along its inner surface and provided only with external corrugations 33' by means of a helical reinforcement element 36' wound around the outside of the tube. This construction produces one continuous, helical air pocket extending along the tubing 3'.

Another example is illustrated in Figure 4 which has an inner tube 32'' of the same kind as that in the arrangement of Figure 2 but has an external layer 34'' of an insulating foam or similar material. The layer 34'' may be held in place by its resilience, being stretched radially to apply over the inner tube 32'' and allowed to shrink back towards its natural diameter. Alternatively, an outer layer 37'' of a heat shrink-fit material may be used, which applies an inward compressive force on the underlying foam layer when it is heated. The use of an insulating foam has a disadvantage because its opacity prevents the clinician seeing the inside of the tubing.

## Claims

1. Tubing having a first end adapted for connection to humidifier means and a second end adapted for connection to a patient breathing device such as a tube or face mask, the tubing comprising a tube that is corrugated externally, characterised in that the tubing (3) includes an outer tube (34) that extends about the corrugated inner tube (32) to trap air between the corrugations (33) between the inner and outer tubes (33 and 34) and thereby thermally insulate the tubing (3) to reduce condensation of humidified gas flowing along the inner tube (32).

2. Tubing according to Claim 1, characterised in that the inner tube (32) is corrugated both externally and internally.

3. Tubing according to Claim 1 or 2, characterised in that the corrugations (33) are annular so that air trapped in recesses between adjacent pairs of corrugations is isolated from each other.

4. Tubing according to any one of the preceding claims, characterised in that the outer tube (34) is uncorrugated.

5. Tubing according to any one of the preceding claims, characterised in that the inner and outer tubes (32 and 34) are transparent.

6. Tubing according to any one of the preceding claims, characterised in that the outer tube (34) is bonded to the inner tube (32) where it contacts the corrugations (33).
